# EUROPEAN PATENT APPLICATION

(11) **EP 3 456 261 A1**
(43) Date of publication of application: **20.03.2019**
(21) Application number: 17201448.2
(22) Date of filing: 13.11.2017
(51) Int. Cl.: A61B 6/00, A61B 5/00, A61B 90/96, A61B 90/00, A61B 90/35, A61B 6/10, A61B 5/11, F16M 13/00, G01N 23/04, G03B 17/56, H01J 35/00, H04N 7/00

(54) **VIDEO AND ACCESSORY APPARATUS FOR A VIDEOFLUOROSCOPY UNIT**

(30) Priority: 18.09.2017 US 201715707123
(71) Applicant: Spine Injury Solutions, Inc., Houston, TX 77007 (US)
(72) Inventor: Spencer, Jon David, League City, Texas 77573 (US)
(74) Representative: Tomkins & Co

(57) **Abstract**

A video fluoroscopy device with a video and data management system. More particularly, it relates to a videofluoroscopy accessory device adapted to securely and removably attach to an X-ray head of a fluoroscopy system or device, and systems to manage the operation of the videofluoroscopy accessory device.

## Description

### CROSS-REFERENCE TO RELATED APPLICATION

This application is based on and claims priority to Provisional Patent Application No. 62/396,281 filed September 19, 2016 entitled "Video and Accessory Apparatus for a Video fluoroscopy Unit", and which is hereby incorporated by reference in its entirety.

### BACKGROUND OF THE INVENTION

### Field of the Invention

The present invention relates to a videofluoroscopy device with a video and data management system. More particularly, it relates to a videofluoroscopy accessory device adapted to securely and removably attach to an X-ray head of a fluoroscopy system or device, and systems to manage the operation of the videofluoroscopy accessory device.

### Description of Related Art

Videofluoroscopy devices are known in the prior art and are most commonly used by doctors and practitioners in the fields of chiropractic, orthopedic, osteopathic and sports medicine. The videofluoroscopy device allows a doctor or practitioner to assess problems or abnormalities of a patient's joints, muscles or bones by recording a real time x-ray video image of such joints, muscles, and bones on a monitor or video display. The video can be reviewed at the doctor's or practitioner's leisure or in real time, allowing for careful and precise evaluation of a particular problem which might not be evident from an external exam or from conventional static x-ray photos.

Most videofluoroscopy devices are relatively small in design and mount to a wall. The device may be used in an office setting or in a mobile medical vehicle. Most typically the videofluoroscopy device mainly consists of a vertical housing enclosing a movement mechanism, the movement mechanism operating a c-arm, the c-arm mounted perpendicularly to a longitudinal axis of the vertical housing and supporting an x-ray device. The movement mechanism directs the c-arm upward and downward along the longitudinal axis of the vertical housing. The x-ray device supported upon the c-arm transmits a real time x-ray image to a video tape recorder thereby recording the movement of the entire body of a patient. In particular, the device records x-ray images of the joints, muscles, and bones and their corresponding movements.

Although videofluoroscopy devices and systems of the prior art have been successful in rendering real time x-ray images of individuals, they do not permit the x-ray viewer to simultaneously view the x-ray and a concurrent video image of the patient and/or the concurrent audio of the patient and/or the videofluoroscopy environment. A need exists for doctors and others to simultaneously view the video x-ray with an external image and sounds of a patient. There is also a need for a videofluoroscopy accessory for accomplishing the above and to allow doctors and others to mount lighting, cameras, microphones, and other medical equipment/tools for optimizing the surgical environment.

### SUMMARY OF THE INVENTION

Provided herein is a videofluoroscopy accessory device adapted to securely and removably attach to an X-ray head of a fluoroscopy system or device. The videofluoroscopy accessory device on the current invention provides a platform for enhancing and supplementing the surgical environment with additional accessories. The accessories that could be used with the present invention include but are not limited to lights, cameras, microphones, and other medical equipment/tools for optimizing the surgical environment.

The present invention can be particularly helpful for allowing for the positioning of a plurality of video cameras around the surgical field when a fluoroscopy system is being used during surgery. This arrangement allows a surgeon or physician to document and/or view a surgical and/or sterile procedure in a hands free manner, thus aiding in maintaining a sterile surgical environment. The height and angle of the accessories, including video cameras, can be adjusted to allow said accessories to move circumferentially around the sterile field while the present invention is mounted on a fluoroscopy system or device.

It is envisioned that the present invention in one embodiment may comprise a plurality of video cameras, each positioned to view a different angle of a medical procedure where the plurality of views are shown with a view of an X-ray image or an X-ray video on a common visual display. The arrangement has been found to aid users in viewing and perceiving a multi-dimensional environment. For example, a display showing four videos simultaneously of three video camera views from different angles and an X-ray video of the same subject area, a user could see 360 degrees about the subject area and within the area via the X-ray video. Accordingly, a person skilled in the art should appreciate the advantages of the present invention.

In one embodiment the present invention is a videofluoroscopy accessory for use with a videofluoroscopy device or system, the videofluoroscopy accessory including a clamp adapted to receive an X-ray head of a videofluoroscopy device, at least one predetermined accessory, at least one accessory mount disposed on the clamp adapted to removably receive at least one predetermined accessory, a computer configured to record information from at least one predetermined accessory and the videofluoroscopy device or system, an electrically isolated connection configured to communicate information from the at least one predetermined accessory and the videofluoroscopy device or system to the computer, and wherein the at least one predetermined accessory is chosen from a group consisting of a camera, a video camera, a light fixture, a microphone, an image projector, a video projector and an imaging device. In another embodiment the invention includes a barcode scanner configured to read a barcode and output data to the computer to be recorded by the computer. In another embodiment of the invention, the circumference of the clamp is adjustable. In another embodiment the invention includes an accessory mount arm adapted to connect the at least one predetermined accessory to the clamp, wherein the accessory mount arm defines a hollow channel for housing a wire or a cable. In another embodiment the invention includes an accessory mount arm adapted to connect the at least one predetermined accessory to the clamp. In another embodiment the invention includes an accessory mount arm adapted to connect the at least one predetermined accessory to the clamp, wherein the accessory mount arm defines a hollow channel for housing a wire or a cable, and further includes means for electrically connecting the at least one predetermined accessory to the videofluoroscopy device. In another embodiment of the invention the electrically isolated connection comprises at least one power over Ethernet connection.

In one embodiment the present invention is a videofluoroscopy accessory for use with a videofluoroscopy device or system, the videofluoroscopy accessory including a clamp adapted to receive an X-ray head of a videofluoroscopy device, at least one predetermined accessory, at least one accessory mount circumferentially disposed on the clamp adapted to removably receive at least one predetermined accessory, a computer configured to record information from at least one predetermined accessory and the videofluoroscopy device or system, at least one accessory mount arm adapted to connect the at least one predetermined accessory to the at least one accessory mount disposed on the clamp, an electrically isolated connection configured to communicate information from the at least one predetermined accessory and the videofluoroscopy device or system to the computer, and wherein the at least one predetermined accessory is chosen from a group consisting of a camera, a video camera, a light fixture, a microphone, an image projector, a video projector and an imaging device. In another embodiment of the invention the circumference of the clamp is adjustable. In another embodiment of the invention the at least one accessory mount arm defines a hollow channel for housing a wire or a cable. In another embodiment the invention includes a barcode scanner configured to read a barcode and output data to the computer to be recorded by the computer. In another embodiment of the invention the at least one accessory mount arm defines a hollow channel for housing at least part of the means for communicating information between the at least one predetermined accessory and the videofluoroscopy device. In another embodiment of the invention the electrically isolated connection comprises at least one power over Ethernet connection. In another embodiment of the invention the at least one accessory mount can be selectively positioned so that the accessory can face any direction. In another embodiment of the invention the at least one accessory mount is motorized and remotely operable from the computer.

Although the invention is described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the spirit and scope of the appended claims. For example, it is to be understood that the present invention contemplates that, to the extent possible, one or more features of any embodiment can be combined with one or more features of any other embodiment.

### BRIEF DESCRIPTION OF THE DRAWINGS

FIG. 1 is a overall schematic showing a videofluoroscopy accessory device system according to an embodiment of the present invention coupled to a typical fluoroscope system;
FIG. 2 is a schematic showing the components of a videofluoroscopy accessory device system according to an embodiment of the present invention of FIG. 1;
FIG. 3 is a perspective view showing a videofluoroscopy accessory device according to an embodiment of the present invention;
FIG. 4 is a top plan view showing a videofluoroscopy accessory device according to one embodiment of the videofluoroscopy accessory device of FIG. 3;
FIG. 5 is a front side elevation view of a videofluoroscopy accessory device according to one embodiment of the videofluoroscopy accessory device of FIG. 3;
FIG. 6 is a left side elevation view of a videofluoroscopy accessory device according to one embodiment of the videofluoroscopy accessory device of FIG. 3;
FIG. 7 is a perspective view of a videofluoroscopy accessory device with the clamp in an open position according to one embodiment of the videofluoroscopy accessory device of FIG. 3;
FIG. 8 is a close up exploded view the parts of the clamp closure according to one embodiment of the videofluoroscopy accessory device of FIG. 3;
FIG. 9 is a close up exploded view showing the parts that make up the electrical enclosure according to one embodiment of the videofluoroscopy accessory device of FIG. 3;
FIG. 10 is a close-up exploded view of a camera mount and a video camera according to one embodiment of the videofluoroscopy accessory device of FIG. 3;
FIG. 11 is an exploded view of an accessory mount arm according to one embodiment of the videofluoroscopy accessory device of FIG. 3;
FIG. 12 is a partial exploded view of a hinge for connecting the electrical enclosure portion to the right side portion of the videofluoroscopy accessory device according to one embodiment of the videofluoroscopy accessory device of FIG. 3;
FIG. 13 is a perspective view showing a videofluoroscopy accessory device fitted to an X-ray head of a fluoroscopy system according to one embodiment of the videofluoroscopy accessory device of FIG. 3; and
FIG. 14 is a front side elevation view of a videofluoroscopy accessory device fitted to an X-ray head of a fluoroscopy system according to one embodiment of the videofluoroscopy accessory device of FIG. 3.

### BRIEF DESCRIPTION OF THE INVENTION

The present invention provides for a videofluoroscopy accessory device adapted to securely and removably attach to an X-ray head of a fluoroscopy system or device. The videofluoroscopy accessory device on the current invention provides a platform for enhancing and supplementing the surgical environment with additional accessories.

These and other features and characteristics of the present invention, as well as the methods of operation and functions of the related elements of structures and the combination of parts and economies of manufacture, will become more apparent upon consideration of the following description with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention. As used in the specification, the singular form of "a", "an", and "the" include plural referents unless the context clearly dictates otherwise.

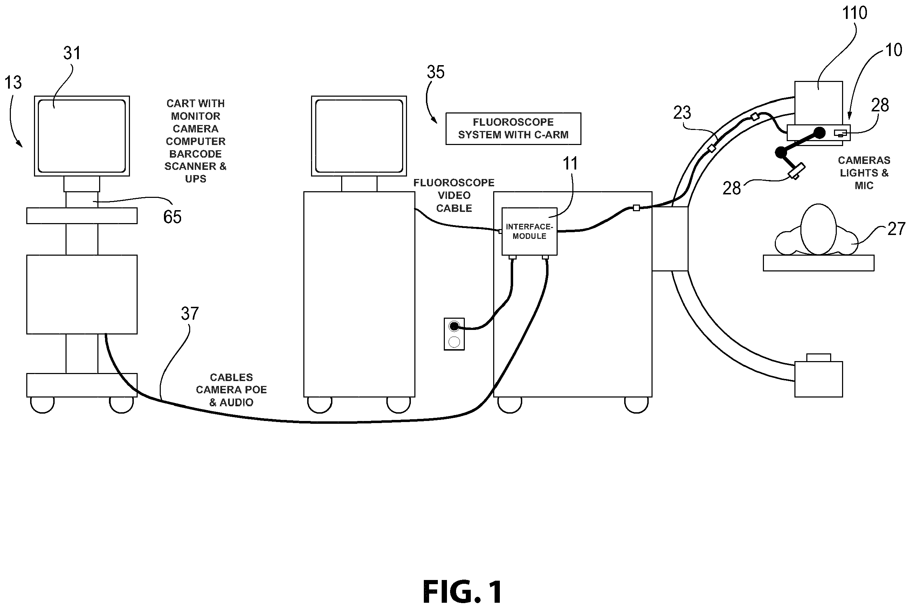

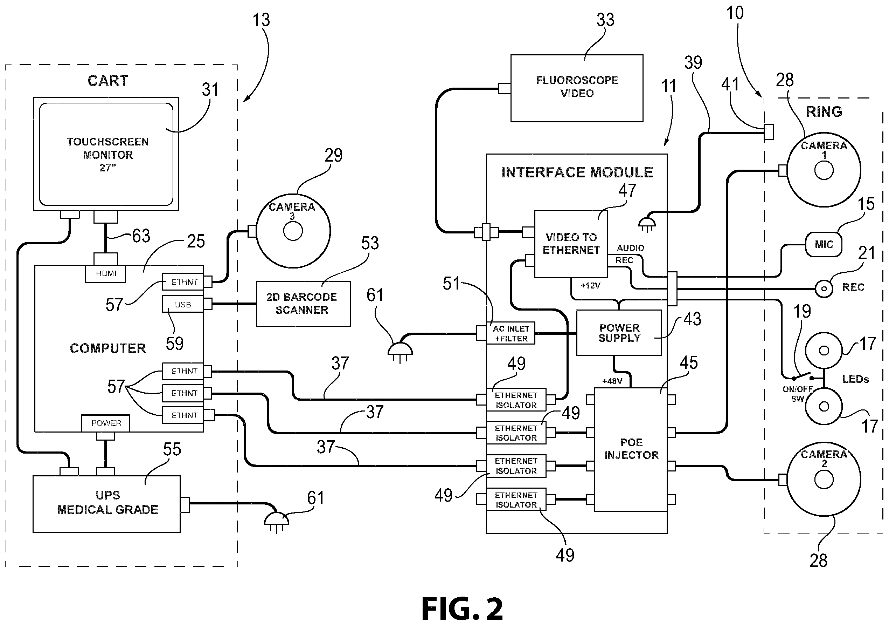

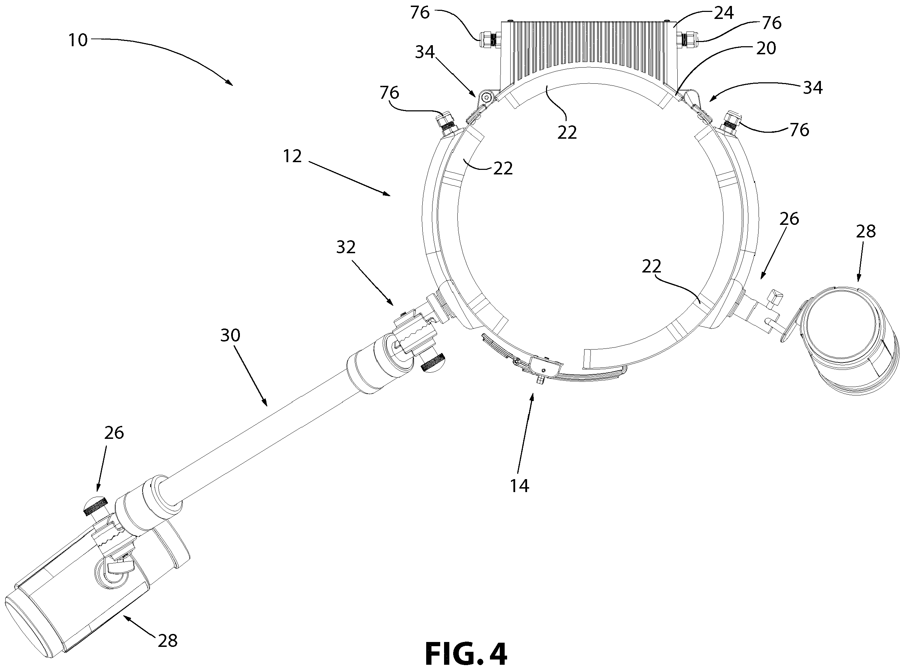

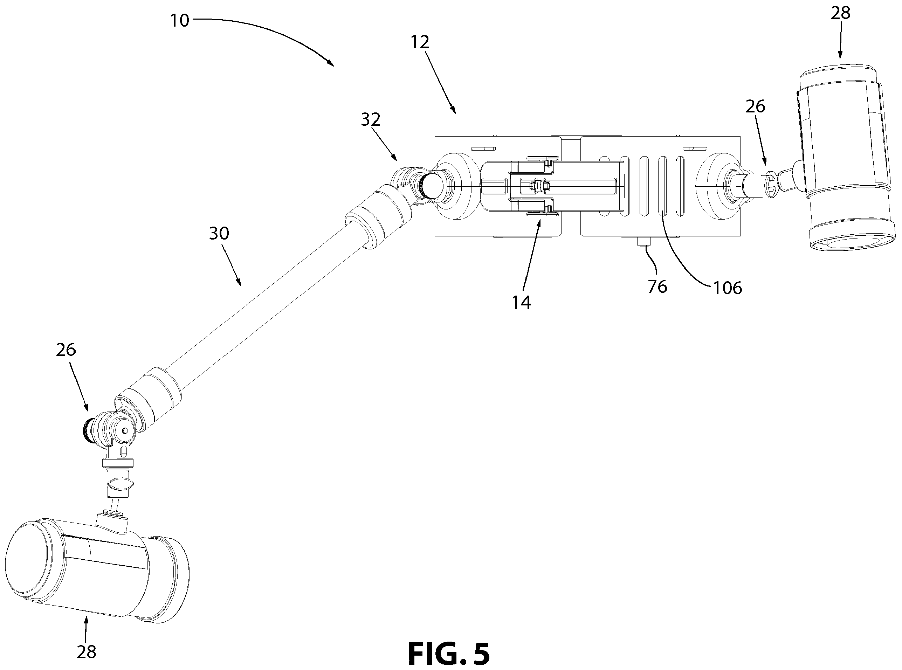

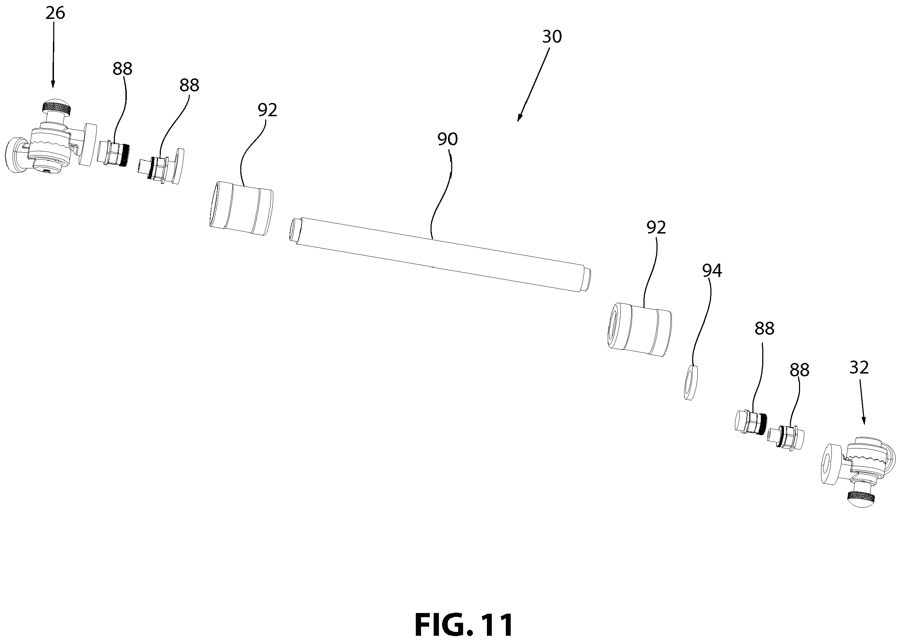

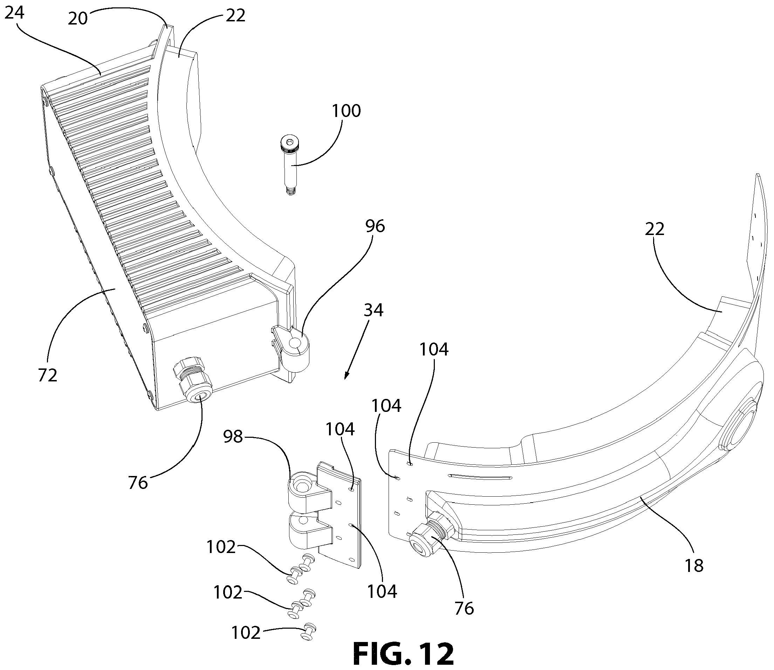

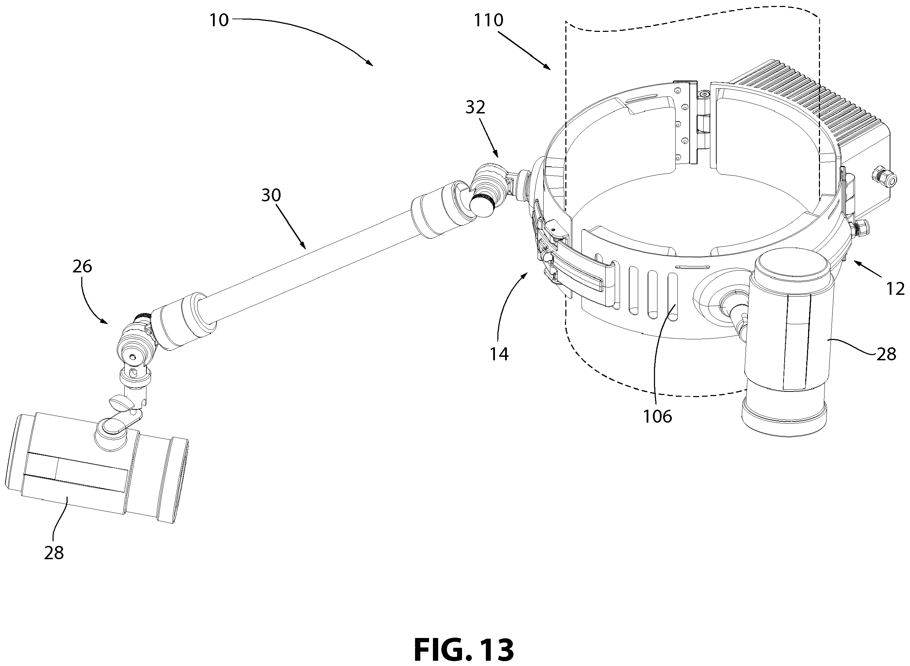

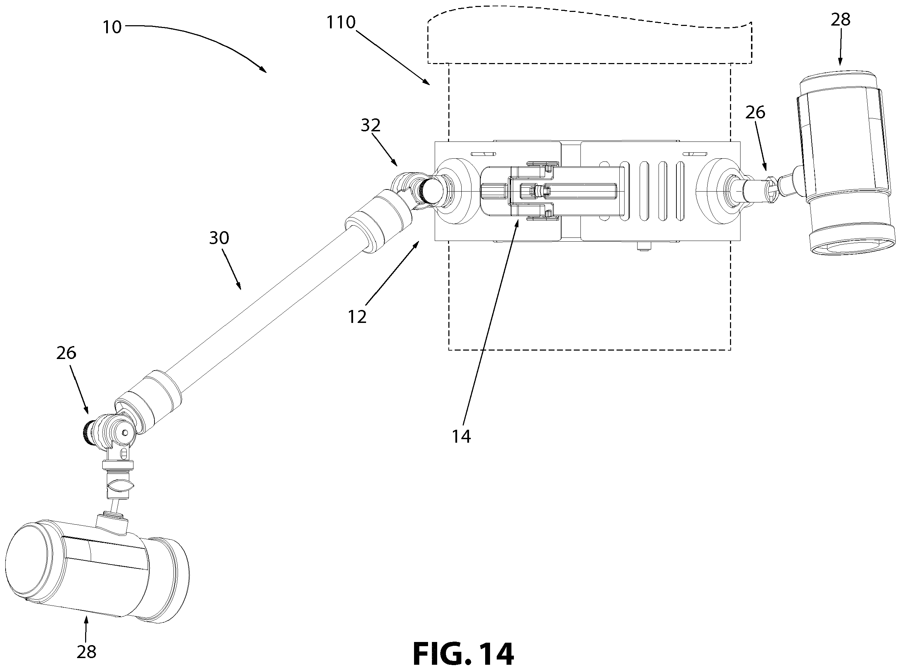

With reference to FIGS. 1-14, the system of the present invention includes a videofluoroscopy accessory device 10, an interface module 11 and a cart 13. The videofluoroscopy accessory device 10 includes a microphone 15 for recording audio during a procedure/surgery, two white LED spotlights 17 for lighting, an on/off switch 19 to control the lights 17, video cameras 28, and a blue LED light bar 21 to indicate recording on/off.

The electronics of the videofluoroscopy accessory device 10 are connected to the interface module 11 via a shielded multiconductor cable 23. The video streams from video cameras 28 along with audio streams from microphone 15 are interfaced to a specialized computer 25 in the cart 13 for video and audio recording. The two cameras 28 and microphone 15 are attached to the videofluoroscopy accessory device 10 near the patient 27 for recording the patient during a procedure. A third video camera 29 is attached to the cart 13 for recording the patient 27 for identification purposes and for recording the overall medical procedure. The monitor 31 on the cart 13 is a touch screen display that shows the video streams. The Interface Module 11 electrically isolates the videofluoroscopy accessory device 10 and x-ray video signals from the cart 13 for electrical safety purposes. The interface module 11 also powers the cameras and lights and the video encoder 33 from the fluoroscope system 35. The video signals are transmitted from the interface module 11 to the cart 13 via Ethernet cables 37. The videofluoroscopy accessory device 10 has a grounding strap 39 that connects to the interface module 11 via a banana plug 41. This feature prevents exposed metal on the videofluoroscopy accessory device 10 from potentially becoming an electrical hazard.

The interface module 11 contains a medical grade power supply 43, a four-port Power-over-Ethernet (PoE) injector 45, a video encoder 47, four medical grade Ethernet isolators 49, and a medical grade power inlet module 51 with fuses. Ports on the PoE injector 45 and Ethernet isolation 49 ports are connected in sequence and allow for three PoE devices to be connected and electrically isolated through the interface module 11. This embodiment of the present invention currently uses two of the ports to connect the video cameras 28 in the videofluoroscopy accessory device 10 to the cart 13. The video encoder 47 receives video from the X-ray head 110 and audio from the microphone in the videofluoroscopy accessory device 10 via the shielded multiconductor cable 23. Video encoder 47 then converts them to a video stream which is output through Ethernet isolators 49. The above features of the interface module 11 prevent current surges from harming the patient 27 or any nearby persons.

The cart includes 13 a touch screen monitor 31, a computer 25, a video camera 29, a barcode scanner 53, an uninterruptable power supply (UPS) 55, four Ethernet receptacles 57, universal serial bus (USB) port 59, and a power receptacle 61. The computer 25 in the cart 13 is specialized for video recording and contains a hard drive which is capable of storing at least 1500 hours of video. The touch screen monitor 31 provides the user with an interface to operate the recording software to view the video streams in real-time and start recording sessions. The touch screen monitor 31 is connected to the computer via video cable with ferrites 63, USB 3.0 cable (not shown), and stereo cable (not shown).

The video camera 29 clips to the touch screen monitor 31 and provides an overall view of the operating room during the procedure/surgery. The video camera 29 is attached to the computer 25 via Ethernet receptacle 57 using Power-over-Ethernet (PoE).

The barcode scanner 53 is attached to the computer 25 via USB port 59. The barcode scanner 53 is mounted to the monitor stand 65 and allows the user to scan the user's or patient's driver's license to document their identity for the procedure/surgery. This documentation of identity can also be used to check that the patient is set to receive the intended treatment, and to prevent similar errors and mistakes.

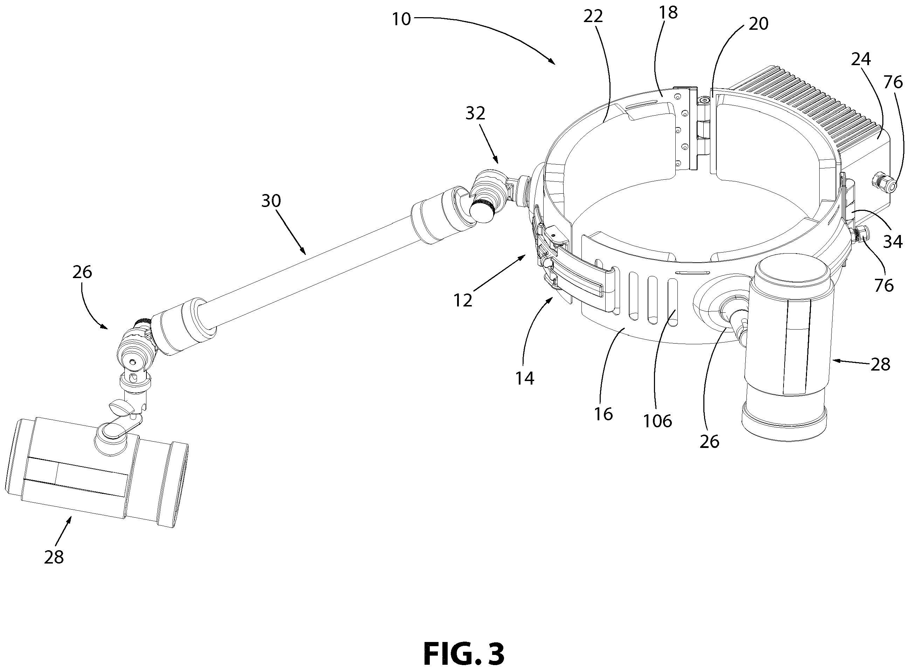

With reference to FIGS. 3-14, the videofluoroscopy accessory device 10 includes a clamp 12 adapted to engage the periphery of an X-ray head 110 of a fluoroscopy system or other X-ray head. The clamp 12 could be a band clamp, or any suitable clamp capable of being secured to an X-ray head 110. X-ray heads are produced in a variety of sizes and shapes and accordingly the clamp 12 should be constructed to fit the applicable X-ray head. X-ray heads are typically cylindrical or cuboidal in shape but could be constructed with almost any shape.

The clamp 12 may further include a closure 14, a left perimeter section 16, a right perimeter section 18, and a back perimeter section 20. The perimeter sections are connected by hinges 34 and are closed together by closure 14. The left perimeter section 16, the right perimeter section 18, and the back perimeter section 20 may have padding 22 or some vibration damping material disposed on the inside perimeter of the clamp 12 to reduce vibration between the x-ray head and the videofluoroscopy accessory device 10. The padding 22 could also be adapted to provide improved fit and securement to the X-ray head 110. The clamp 12, particularly left perimeter section 16, right perimeter section 18, and back perimeter section 20, may be constructed of a rigid, semi-rigid, flexible material or a combination thereof.

The clamp 12 may further include an electrical enclosure 24 for containing electrical components and connections to the interface module 11. It is envisioned in other embodiments of the present invention that the electrical enclosure 24 could contain power and communication means for powering any accessories and providing communication between the accessories and a videofluoroscopy system or the like. For example, the power and communication means could comprise a networking switch, a circuit board, a power over Ethernet (POE) system or device, a wireless communication system, a battery, a power supply, a fuse, a breaker, a microprocessor, a power isolation system or any components contained in the interface module 11 described herein.

The clamp 12 further includes at least one accessory mount 26 for mounting an accessory, such as, video camera 28. The clamp 12 may also include an arm mount 32 for attaching an accessory mount arm 30 and an accessory mount 26 to the clamp 12.

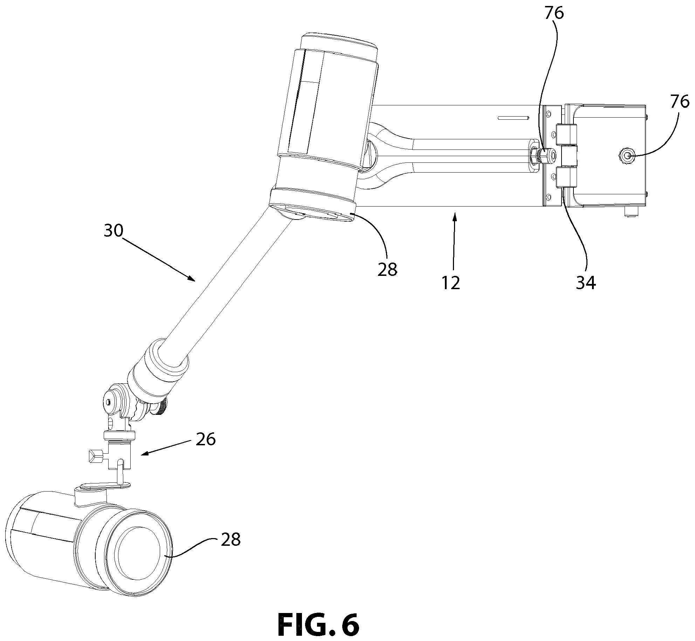

Now referencing FIG. 6, the closure 14 comprises a bracket 36 for connecting the closure to the clamp 12. Apertures 38 in bracket 36 receive short pins 40 and short pins 40 are received in apertures 42 of paddle 44. Long pin 46 is received in apertures 48 of the paddle 44 and aperture 50 of hook member 52. Rotatable tab 54 is rotatably connected to bracket 36 at connection 56. Rotatable tab 54 is configured to be received in tab hole 58 and to lock the closure closed when rotated in tab hole 58. The bracket 36 may be secured to clamp 12 by means of screw 60 and spring 62 disposed on the bracket 36.

Now referencing FIGS. 1, 3, 5-6 and 11-12, hook member 52 of closure 14 is configured to engage a plurality of slots 106 in the left perimeter section 16 of the clamp 12.

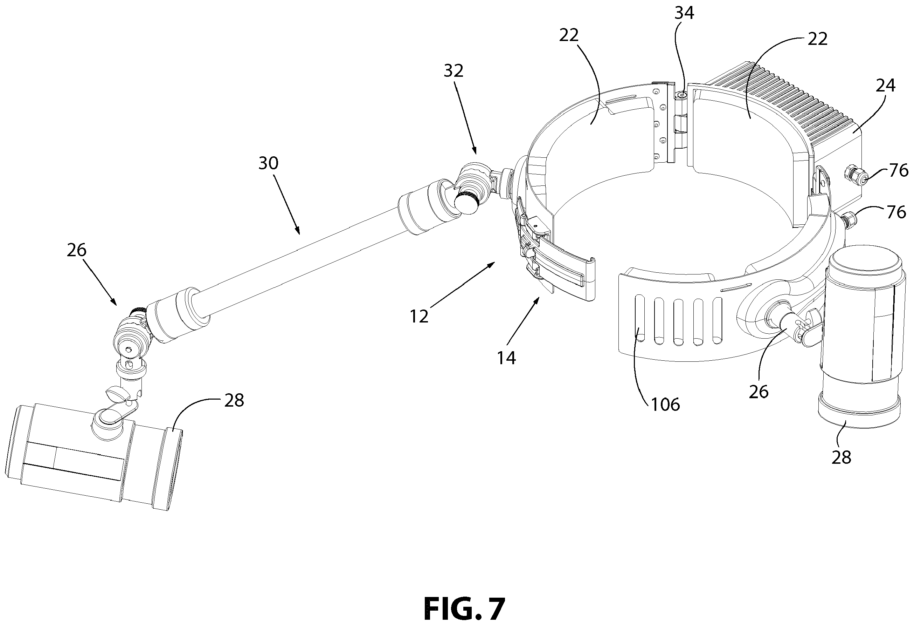

Now referencing FIG. 7, the electrical enclosure 24 and back perimeter section 20 may be integrally formed as shown or could be separately formed. As discussed above padding 22 may be attached to the clamp 12 to dampen vibrations or assist in forming a secure attachment to an X-ray head. The electrical enclosure 24 may include sub-enclosure 64 which could be attached by means of screws 66 to the inside of electrical enclosure 24. The sub-enclosure may be transparent or translucent and contain a light source for indicating that the videofluoroscopy accessory device 10 is powered on or to indicate some other status of the device. Screw holes 68 are provided in order to secure first face plate 70 and second face plate 72 to the electrical enclosure 24 by means of screws 74. The electrical enclosure 24 may also include electrical connectors 76 for routing wires or cables to any desired accessory.

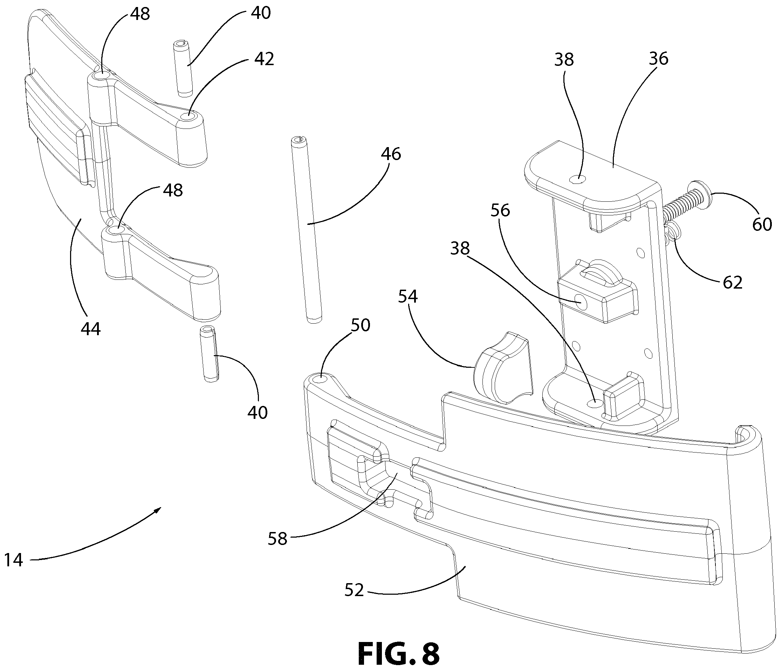

Now referencing FIG. 8, the accessory mount 26 may include first rotatable joint element 78 and second rotatable joint element 80 joined by hand screw 82, which are joined to rotatable mount 84 by means of connector 86. The accessory mount 26 may then be connected to the clamp 23 or the accessory mount arm 30 by connector 88. The accessory mount 26 could be any suitable adjustable camera mount or other mount for attaching an accessory. Preferably the accessory mount can be selectively positioned so that the accessory can face any direction. This can be accomplished with suitable lockable ball and socket joint or a combination joint that includes a plurality of rotatabable joints arranged so that the accessory can be oriented in any direction. It is also envisioned that the accessory mount could be motorized and remotely operable.

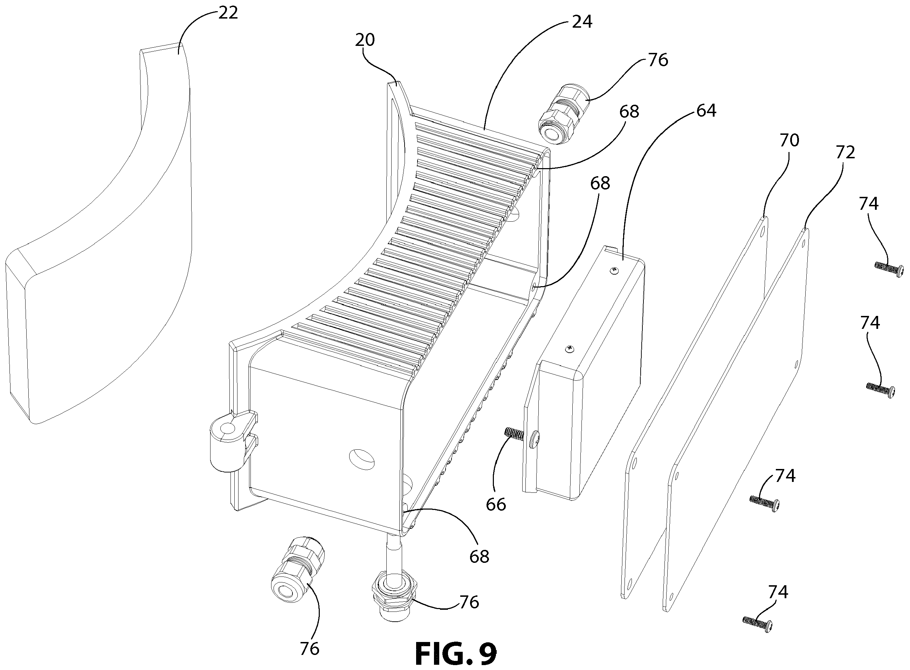

Now referencing FIG. 9, the accessory mount arm 30 may include arm 90 with couplings 92 which may be connected to accessory mount 26 and/or arm mount 32 by means of connectors 88 and washer 94. The accessory mount arm 30 may be hollow and may have a channel or course inside to house wiring or cabling for connecting accessories to the electrical enclosure 24.

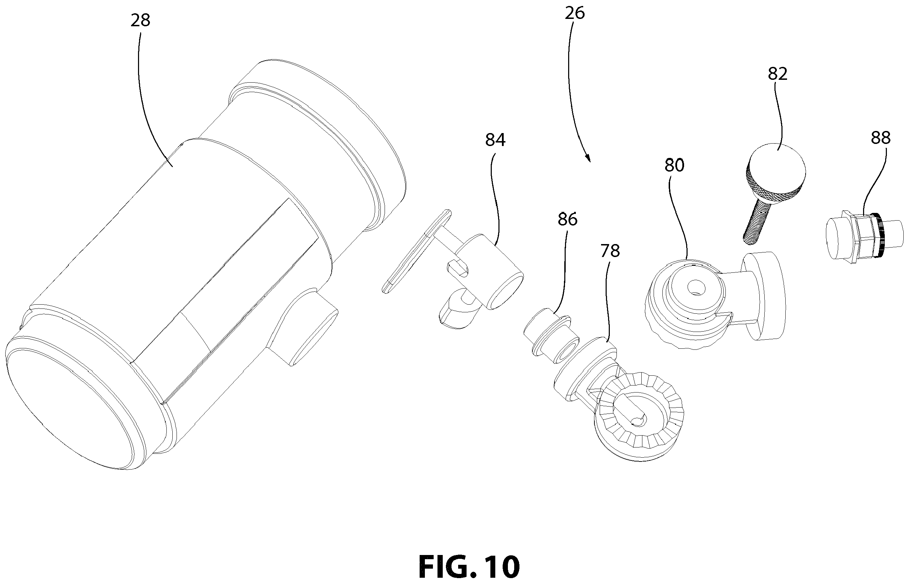

Now referencing FIG. 10, the right perimeter section 18 and the back perimeter section 20 may be hingedly connected to each other by hinge 34. Hinge 34 may comprise first barrel 96, second barrel 98 and pin 100 for connecting both barrels to each other. The first barrel 96 may be integrally formed with back perimeter section 20. The hinge 34 may then be connected to right perimeter section 18 by fastening fasteners 102 through holes 104.

Although the invention is described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the spirit and scope of the appended claims. For example, it is to be understood that the present invention contemplates that, to the extent possible, one or more features of any embodiment can be combined with one or more features of any other embodiment.

## Claims

1. A videofluoroscopy accessory (10) for use with a videofluoroscopy device or system (35), the videofluoroscopy accessory (10) comprising:
a clamp (12) adapted to receive an X-ray head (110) of a videofluoroscopy device (35),
at least one predetermined accessory (15, 17, 21, 28),
at least one accessory mount (26) disposed on the clamp (12) adapted to removably receive at least one predetermined accessory (15, 17, 21, 28),
a computer (25) configured to record information from at least one predetermined accessory (15, 17, 21, 28) and the videofluoroscopy device or system (35),
an electrically isolated connection (49) configured to communicate information from the at least one predetermined accessory (15, 17, 21, 28) and the videofluoroscopy device or system (35) to the computer (25), and
wherein the at least one predetermined accessory is chosen from a group consisting of a camera (28), a video camera (28), a light fixture (17, 21), a microphone (15), an image projector (17, 21), a video projector (17, 21) and an imaging device (28).

2. The videofluoroscopy accessory (10) according to claim 1, further comprising a barcode scanner (53) configured to read a barcode and output data to the computer (25) to be recorded by the computer (25).

3. The videofluoroscopy accessory (10) according to claim 1, wherein the circumference of the clamp (12) is adjustable.

4. The videofluoroscopy accessory (10) according to claim 1, further comprising an accessory mount arm (30, 90) adapted to connect the at least one predetermined accessory (15, 17, 21, 28) to the clamp (12), wherein the accessory mount arm (30, 90) defines a hollow channel for housing a wire or a cable (23).

5. The videofluoroscopy accessory (10) according to claim 1, further comprising an accessory mount arm (30, 90) adapted to connect the at least one predetermined accessory (15, 17, 21, 28) to the clamp (12).

6. The videofluoroscopy accessory (10) according to claim 1, further comprising an accessory mount arm (30, 90) adapted to connect the at least one predetermined accessory (15, 17, 21, 28) to the clamp (12), wherein the accessory mount arm (30, 90) defines a hollow channel for housing a wire or a cable (23), and
further comprising means for electrically connecting (43,45) the at least one predetermined accessory (15, 17, 21, 28) to the videofluoroscopy device (35).

7. The videofluoroscopy accessory (10) according to claim 1, wherein the electrically isolated connection (49) comprises at least one power over Ethernet connection (49, 45).

8. A videofluoroscopy accessory (10) for use with a videofluoroscopy device or system (35), the videofluoroscopy accessory (10) comprising:
a clamp (12) adapted to receive an X-ray head (110) of a videofluoroscopy device (35),
at least one predetermined accessory (15, 17, 21, 28),
at least one accessory mount (26) circumferentially disposed on the clamp (12) adapted to removably receive at least one predetermined accessory (15, 17, 21, 28),
a computer (25) configured to record information from at least one predetermined accessory (15, 17, 21, 28) and the videofluoroscopy device or system (35),
at least one accessory mount arm (30, 90) adapted to connect the at least one predetermined accessory (15, 17, 21, 28) to the at least one accessory mount (26) disposed on the clamp (12),
an electrically isolated connection (49) configured to communicate information from the at least one predetermined accessory (15, 17, 21, 28) and the videofluoroscopy device or system (35) to the computer (25), and
wherein the at least one predetermined accessory (15, 17, 21, 28) is chosen from a group consisting of a camera (28), a video camera (28), a light fixture (17, 21), a microphone (15), an image projector (17, 21), a video projector (17, 21) and an imaging device (28).

9. The videofluoroscopy accessory (10) according to claim 8, wherein the circumference of the clamp (12) is adjustable.

10. The videofluoroscopy accessory (10) according to claim 8, wherein the at least one accessory mount arm (30, 90) defines a hollow channel for housing a wire or a cable (23).

11. The videofluoroscopy accessory (10) according to claim 8, further comprising a barcode scanner (53) configured to read a barcode and output data to the computer (25) to be recorded by the computer (25).

12. The videofluoroscopy accessory (10) according to claim 8, wherein the at least one accessory mount arm (30, 90) defines a hollow channel for housing at least part of the means for communicating information (23, 43) between the at least one predetermined accessory (15, 17, 21, 28) and the videofluoroscopy device.

13. The videofluoroscopy accessory (10) according to claim 8, wherein the electrically isolated connection (49) comprises at least one power over Ethernet connection (49, 45).

14. The videofluoroscopy accessory (10) according to claim 8, wherein the at least one accessory mount (26) can be selectively positioned so that the accessory (15, 17, 21, 28) can face any direction.

15. The videofluoroscopy accessory (10) according to claim 8, wherein the at least one accessory mount (26) is motorized and remotely operable from the computer (25).
